# EUROPEAN PATENT SPECIFICATION

(11) **EP 3 675 941 B1**
(45) Date of publication and mention of the grant of the patent: **21.06.2023**
(21) Application number: 18783095.5
(22) Date of filing: 31.08.2018
(51) Int. Cl.: A61M 15/00, A61M 11/00

(54) **AN INHALER FOR A SINGLE-DOSE OF DRY POWDER AND A METHOD FOR ADJUSTING AN INHALER FOR SINGLE-DOSE OF DRY POWDER FOR DELIVERING A SPECIFIC MEDICAMENT**
INHALATOR FÜR EINE TROCKENPULVER-EINZELDOSIS UND VERFAHREN ZUR EINSTELLUNG EINES INHALATORS FÜR EINE TROCKENPULVER-EINZELDOSIS ZUR ABGABE EINES SPEZIFISCHEN MEDIKAMENTS
INHALATEUR POUR UNE DOSE UNIQUE DE POUDRE SÈCHE ET PROCÉDÉ DE RÉGLAGE D'UN INHALATEUR POUR UNE DOSE UNIQUE DE POUDRE SÈCHE POUR ADMINISTRER UN MÉDICAMENT SPÉCIFIQUE

(30) Priority: 31.08.2017 PL 42271617
(43) Date of publication of application: 08.07.2020
(73) Proprietor: Pulinno Sp. Z o.o., 85-862 Bydgoszcz (PL)
(72) Inventor: ROSZCZYK, Pawel, Warszawa (PL); SOSNOWSKI, Tomasz, Warszawa (PL); MOSKAL, Arkadiusz, 05-250 Radzymin (PL); KLUCZ, Emil, 88-400 Znin (PL); RATAJCZAK, Radoslaw, 85-088 Bydgoszcz (PL); ZEBROWSKI, Pawel, 71-664 Szczecin (PL); SIERACKI, Filip, 85-435 Bydgoszcz (PL); CICHOSZ, Anna, 85-565 Bydgoszcz (PL); MATULEWICZ, Karolina, 66-400 Gorzow Wielkopolski (PL); SWIETLIK, Daria, 85-042 Bydgoszcz (PL); SIEROSLAWSKA, Anna, 85-088 Bydgoszcz (PL); MROZ, Paulina, 85-028 Bydgoszcz (PL); SICHEL, Joanna, 89-200 Szubin (PL); RYCHLAWSKI, Krzysztof, 86-014 Sicienko (PL); KLUCZ, Krzysztof, 86-005 Biale Blota (PL); WIRWICKI, Mateusz, 85-137 Bydgoszcz (PL); WISNIEWSKI, Waldemar, 85-704 Bydgoszcz (PL)
(74) Representative: Patpol Kancelaria Patentowa Sp. z o.o.
(86) International application number: PCT/IB2018/056672
(87) International publication number: WO 2019/043639

(56) References cited:
- WO-A1-2014/058208
- JP-B2- 3 011 898
- US-A1- 2010 275 917

## Description

### FIELD OF THE INVENTION

The present invention relates to a single-dose dry powder inhaler, especially for capsules containing a medicine.

The present invention further relates to a universal inhaler for administering a single dose of a dry powder, wherein the inhaler is adaptable for use with different dry powders. The present invention further relates to a method for adjusting a universal single-dose dry powder inhaler depending on the physical properties of such a powder.

### BACKGROUND OF THE INVENTION

The effectiveness of an inhalation therapy depends on the appropriate deposition of an inhalation agent in a patient's airways (Sosnowski Tadeusz R., Aerozole Wziewne i Inhalatory, Politechnika Warszawska, Warszawa 2012, ISBN 978-83-906658-7-0). The smaller the particles of a medicinal substance suspended in the inhaled aerosol, the farther they are deposited. Distribution of particles is as follows: particles having diameter less than 10 µm are deposited in a patient's upper airways, particles having diameter in the range from 5 to 10 µm are deposited in the bronchi, whereas particles having diameter in the range from 3 to 4 µm are deposited in the bronchioles. Particles having diameter in the range from 1 to 2 µm are deposited in the alveoli.

Since appropriate deposition of a medicinal substance in a patient's airways determines the effectiveness of an inhalation therapy, selection of an active substance and its carrier for the generation of particles having specific parameters is of significance. In the case of a "dry-powder" type of medicine, obtaining a desired particle fraction distribution is the most important factor.

The effectiveness of the process of breaking down the particles of a medicinal product depends on the forces created as a result of a patient's inspiration. Frequently, the force of a patient's inspiration is too small, and the patient fails to assimilate an appropriate dose of an active substance. Therefore, one aims at implementing structural solutions, whereby the force of a patient's inspiration doesn't have to be too big, allowing an appropriate breaking down the particles of a medication while maintaining suitable parameters of the resulting aerosol. Thus, the deposition efficiency depends on the amount of particle fractions smaller than 10 µm contained in a medical powder. In addition to an active substance, a powder contains also a substance being a carrier, *e.g.,* lactose.

### PRIOR ART

Structural solutions of inhalers adapted to administer medicinal inhalation agents using a medicinal substance in the form of a dry powder have been known for many years.

For example, the patent No. US 3991761 published in 1976 discloses an inhaler for administering powdered medicinal products contained in a capsule. The inhaler comprises a body portion having a chamber for placing therein a capsule, piercing devices in the body portion, capable of perforating a capsule in the capsule chamber, and air intake passages communicating with the capsule chamber and arranged in such a manner that air drawn through these passages, when inhaling air through an inhaler passage, leading from the chamber imparts a rotary and shaking motion to a capsule in the capsule chamber. The inhaler is substantially comprised of two main parts, a body portion of the inhaler and a tubular or mouthpiece portion. These two portions are rotatably joined and releasably latched to each other to allow access to the interior of the body portion so as to place a capsule containing the substance for inhalation in a position in the capsule chamber. Following perforation of the capsule by the piercing devices, inhalation by the patient through the tubular or mouthpiece portion causes the powder to be drawn out of the capsule and entrained in the inhaled air.

The air intakes are arranged so that intake air passes tangentially into the capsule chamber, and the capsule chamber has an elongated recess in the bottom wall thereof, in which recess the capsule in the chamber rests when the inhaler is not in use. The air intake is drawn from the recess into the capsule chamber by the air passing through the capsule chamber from the intake passages to the inhalation passage upon inhalation. The body portion and tubular portion are preferably pivotally attached together by means of a pivot pin, the axis of which is parallel to the axis of the tubular portion by which is placed on one side thereof, allowing disengagement or locking by a simple movement of one portion with respect to the other about an axis parallel to the axis of the tubular portion of the inhaler. The piercing devices comprise movable push-buttons, each having a set of elongated spikes arranged to enter a capsule in the recess within the capsule chamber and to effect perforation thereof by pressing a push button. In the preferred embodiment, there are two such push-buttons, each being maintained in a normal position by four independent springs, each being held in place by rims on the body which engage cooperating shoulders on each push button. Each of the push buttons has four elongated metal spikes. Perforation of a capsule in the elongated recess at the bottom of the capsule spinning chamber is thus simply effected by a simple pressing one or both of the push buttons.

The shape of the elongated recess in the bottom wall of the capsule is preferably substantially the same as that of a conventional medication capsule, the recess being generally rectangular with rounded ends. Preferably the spikes or pins of the piercing devices are coaxial with the recess so that a capsule is perforated at its ends. The capsule chamber is preferably circular so that a pierced capsule is caused to rotate solely by the tangential intake air upon inhalation. The intake air also acts to draw the capsule out of the recess upon inhalation so that operation is effectively automatic. The diameter of the capsule chamber is slightly larger than the length of a conventional medication capsule so that there is a small clearance between the chamber and the ends of the capsule as it rotates; causing shaking of the capsule as it strikes against the cylindrical wall of the capsule chamber, thereby urging the powder out from the capsule and causing it to be mixed with the intake air upon inhalation.

The tubular portion of the inhaler consists essentially of a circular pipe having at one end a base having an abutment stop for placing in the inhaler body. The base of the tubular portion forms, when the parts of the inhaler are assembled together, the upper part of the capsule chamber and has a slightly concave surface for facilitating the rotation of a capsule. At the opening of the inhalation passage of the tubular portion there is a grating, suitably dimensioned to optimize the air/powdered substance ratio.

The Polish patent application No. P.412009 discloses a single-dose dry powder inhaler. The essence of this technical solution is use for perforating a capsule of two assemblies of three spikes of 0.8 ± 0.1 mm in diameter, having slanted edges, arranged to form an equilateral triangle. The spikes in the particular assemblies are turned 180° degrees relative to each other. This way, there is provided generation of an aerosol of solid particles in the air, having a fraction of a suitable particle size distribution.

The document No. WO 2005/113042 discloses an inhaler comprising a hinge enabling an articulated mounting the mouthpiece portion to the back edge of the body. This allows to move the mouthpiece portion between an open position for inserting a capsule and a closed position for dosing, about an axis perpendicular to the longitudinal axis of the inhaler, by a user applying a pushing force to the mouthpiece portion. The inhaler body comprises a pair of opposite axial grooves with the hinge axles placed therein, protruding out of the hinge element of the mouthpiece portion.

Examples of similar technical solutions are disclosed in the patent specification Nos. GB1122284, EP0005585, EP1603615, EP2865403 and EP2617451. A common feature of the structure of these inhalers is that they each comprise a first chamber in which a capsule containing an active substance is placed, elements for perforating the capsule and air intake passages, a second, usually oval chamber, in which, upon inhalation by a user, a perforated capsule rotates to release a medicine from the capsule through said perforation, and a mouthpiece with a grating, constituting an air outlet and, upon inhalation, a passage for transporting the medication released from a perforated capsule in the oval chamber.

Patent documents WO2014/058208A1 and JP3011898B2 are hereby acknowledged.

### SUMMARY OF THE INVENTION

The invention is defined by the appended claims.

The solution constituting the subject of the invention is also based on the size of the particles of a medication. Nevertheless, other structural solutions are proposed, which, on one hand, ensure appropriate distribution, so-called "de-aggregation" of a medication into particles smaller than 10 µm, and on the other hand provide universality of the solution, whereby adaptation of the structural parameters allows to use the device with different kinds of medications. The air passage system with adapted structural parameters enables administration of different kinds of active substances.

This objective is attained by means of a single-dose dry powder inhaler, as defined in independent claim 1. Preferable embodiments of said inhaler are defined in claims dependent thereupon.

Another purpose of the invention is to provide a universal technical solution based on the above-described principle, which will be operable within a broad range of self-resistance coefficient, while ensuring delivery of any medication of the required particle diameter distribution, wherein the amount of fraction particles smaller than 10 µm is bigger.

This objective is attained by means of a single dose-dry powder inhaler, as defined in independent claim 4, whereas the preferred embodiments thereof are defined in claims dependent thereupon.

Yet another objective is to provide a method for adjusting a universal inhaler and a method for dosing a specific medication with particle sizes falling within the required scope and with the required self-resistance coefficient of the device.

This objective is attained by means of a method for adjusting a single-dose dry powder inhaler, as defined in independent claim 10, whereas the preferred embodiments thereof are defined in claims dependent thereupon.

### BRIEF DESCRIPTION OF THE FIGURES OF THE DRAWING

The invention will now be described in more detail with reference to the figures of the drawing, wherein:
Fig. 1 is a perspective view of an assembled single-dose dry powder inhaler according to the invention,
Fig. 2 is a perspective view of the inhaler of Fig. 1 with a removed cover,
Fig. 3 is a perspective view of the inhaler of Fig. 1 with a removed cover and an open tube module,
Fig. 4 is a perspective side view of the inhaler of Fig. 3 with a removed cover and an open tube module,
Fig. 5 is an exploded view of the inhaler of Fig. 1,
Fig. 6 is a perspective view of the body of the inhaler of Fig. 5,
Fig. 7 is a top view of the body of the inhaler of Fig. 6,
Fig. 8 is a side view of the body of the inhaler of Fig. 6,
Fig. 9 is a side view of the push-button of Fig. 5,
Fig. 10 is front view of the push-button of Fig. 9,
Fig. 11 is a top view of the capsule chamber of Fig. 5,
Fig. 12 is a side view of the capsule chamber of Fig. 11,
Fig. 13 is a perspective top view of the upper cover of Fig. 5,
Fig. 14 is a perspective bottom view of the upper cover of Fig. 13,
Fig. 15 is a side view of the tube of the mouthpiece of Fig. 5,
Fig. 16 is a longitudinal cross-section of the tube of the mouthpiece of Fig. 15,
Fig. 17 is a side view of the mesh of the mouthpiece of Fig. 5,
Fig. 18 is a side view of the base of the mouthpiece of Fig. 5,
Fig. 19 is a top view of the base of the mouthpiece of Fig. 18,
Fig. 20 is a bottom view of the base of the mouthpiece of Fig. 19,
Fig. 21 is a segment of a cross-section of the base of the mouthpiece of Fig. 20 along the C-C line,
Fig. 22 is a graph of the relationship of the air stream to self-resistance of the inhaler,
Fig. 23 is a graph of distribution of particle sizes for a D3 model at 40 LPM,
Fig. 24 is a graph of distribution of particle sizes for a D1 model at 60 LPM,
Fig. 25 is a graph of distribution of particle sizes for a D1 model at 70 LPM,
Fig. 26 is a graph of distribution of particle sizes for Flutixon at 90 LPM,
Fig. 27 is a diagram of a measurement system for determining self-resistance of an inhaler according to the invention,
Fig. 28 is a graph of self-resistance coefficients of the examined embodiments of an inhaler according to the invention with the self-resistance coefficient values of selected commercial inhalers placed therein,
Fig. 29 shows graphs presenting the impact of the size of the mesh of the mouthpiece on self-resistance coefficient of the inhaler R_{D},
Fig. 30 is a graphic presentation of adjusting an inhaler according to the present invention when in the original inhaler a smaller particle size distribution of a medication is obtained.
Fig. 31 is a graphic presentation of selecting an inhaler according to the present invention when in the original inhaler a bigger particle size distribution of a medication is obtained.

### DETAILED DESCRIPTION OF THE INVENTION

The technical solution according to the invention is also based on the size of the particles of an active substance, however, different structural solutions are proposed, which, on the one hand, ensure a suitable breaking, so-called "de-aggregation" of a medicine into particles having diameter less than 10 µm and, on the other hand, provide a universality of the solution such that adapting the design parameters allows for the device to be used with different kinds of medicines. The air passage system with adapted design parameters enables administration of different kinds of active substances.

Detachment of particles of a medicinal substance from the carrier is effected as a result of a collision of still homogeneous particles of the medicine-carrier aggregates with other particles: i) in the capsule itself by means of generation of negative pressure upon a patient's inspiration, which increases also in the capsule itself as a result of perforating the cover of the capsule; ii) with the walls of the chamber which the capsule with a medicine enters under the pressure upon leaving the basic chamber, wherein, as a result of rotation, a centrifugal force breaks down the particles released from the capsule; iii) with the poles of the mesh that separate the space in which the capsule with a medicine rotates, and the outlet tube in the mouthpiece of the device; iv) with the walls of the tube forming an outlet passage for the inhaled air; v) with the walls of the constriction in the tube forming an outlet passage for the inhaled air.

The first parameter of the device characteristics is its self-resistance coefficient R_{D} (hPa^{0,5}min/dm³) used to classify inhalation devices as low- (R_{D} < 0.07 hPa^{0,5}min/dm³), medium- (R_{D} ranging from 0.07 to 0.1 hPa^{0,5}min/dm³) and high-resistance (R_{D} > 0.1 hPa^{0,5}min/dm³) devices.

The proposed solution in the form of an air passage system having adapted structural parameters allows to adapt the device to a patient's possibilities and needs, as a result of which, it is possible for the device to fall within the low-, medium- or high-resistance range.

Another parameter significant for an inhalation device is the above-mentioned aerosol particle size distribution.

The essence of the invention is the shape and the cross-section area of the air intake passages, structural elements of a modular mouthpiece with an internal constriction, a capsule rotation chamber and a grating with structural parameters in a single-dose dry powder inhaler, adapted for different kinds of a dry powder containing a medicine, in particular, particular components of the modular mouthpiece 21 are interconnected by means of a latching system ensuring durability of the connections. The structural supporting element of the entire system is the body 1, in which the supporting elements are 4 pillars 2 tipped with latches 3 providing a permanent connection with the upper cover 15 by means of a system of special latch spots 18 in the cover 15. The body 1, by means of a special system of ribs 5, is a carrier for a transparent capsule chamber 11. It comprises a capsule chamber 14, in which a capsule is perforated by means of spikes 10 movable in guide holes 13, allowing a powder to be released from the capsule coating during the inhalation process.

Another important feature of the invention is the air passage system in the modular mouthpiece 21. The structural solutions based on the possibility to use different widths of the intake passages 25 and different internal diameter S1 of the tube 21C by means of the internal constrictions 27, which decrease the through diameter S2, allow to adapt the device to the needs of a specific user, whom can be recommended a specific therapy applying this method.

The solution described herein is based on a system of two opposite push-buttons 7 fitted in the device, comprising: lower 9 and upper 8 protrusions, matching the system of lower guides 16 in the body 1 and upper guides 19 in the cover 15, as well as springs 23 allowing depression of the push-buttons 7 following introduction of a spike 10 through a guide hole 13 in the transparent capsule chamber 11 into the capsule chamber 14. The inhaler ensures precise perforation of a capsule, increasing the effectiveness of the therapy and the possibility to use the device multiple times.

The inhaler according to the invention ensures high efficiency of operation of the air passage system by means of tight connections between the particular elements and the entire structure, as well as by means of the precision of the preparatory processes carried out inside the inhaler prior to the proper inhalation stage, such as perforating a capsule.

Precise perforation of a capsule coating by means of specially shaped spikes movable through the guide holes allows proper administration of a therapy and ensures an efficient process of braking the participles into suitable fractions.

It is due to the nature of an ailment or the possibility to inhale harder, that the producers apply different structural solutions that satisfy particular needs. Unfortunately, these solutions are capable of handling only one medication, which, for example, in the case of application of a low-resistance device, does not yield suitable results. Therefore, the practice shows that inhalation devices are selected to match a specific medication. If a patient has to take several different medicines of different effectiveness, a manufacturer produces separate devices for each medicine. The modularity of the described device and its adjustable parameters allow using a single device falling within the medium- and high-resistance range for different treatment types. This opinion is based on an analysis of R_{D} values of the structural solutions matrix (Table 1) relative to the R_{D} values available on the market of commercial inhalers of different resistivity (Table 2).

**Table 1. The matrix of the R_{D} coefficient values obtained for an air passage system having adjustable structural parameters.**

| **The values of the RD coefficient for particular structural modules** | | | | | | |
|---|---|---|---|---|---|---|
| Air intake passage modules 21B | Height to width ratio | | Tube modules 21C with different internal diameter | | | |
| | | | **A** | **B** | **C** | **D** |
| | Height | Width | 0% of coverage | 33% of coverage | 66% of coverage | 85% of coverage |
| **1** | 1 | 0.85 | 0.0777 | 0.0743 | 0.0751 | 0.1074 |
| **2** | 1 | 0.50 | 0.0901 | 0.0901 | 0.0917 | 0.1286 |
| **3** | 1 | 0.25 | 0.1106 | 0.1268 | 0.1254 | 0.1630 |

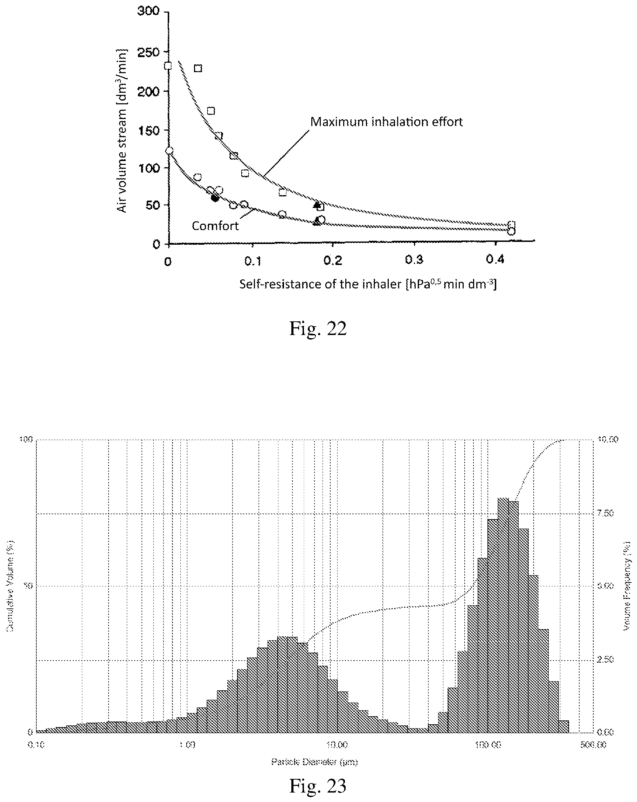

From a patient's point of view, in the process of designing the structure, one needs to take into account the so-called scope of a patient's cooperation with an inhaler, as shown in Fig. 22, presenting the scope of a patient's cooperation with a dry powder inhaler (DPI inhalers).

In the process of designing an air passage system having adjustable structural parameters, it was taken into account that in the case of high-resistance inhalers, it is rarely possible or not possible at all for an ill patient to inhale with a force generating a flow of 100 L/min. The comparative data set (Table 2) contains information about the maximum flow values necessary to arrive at a suitable therapeutic effect.

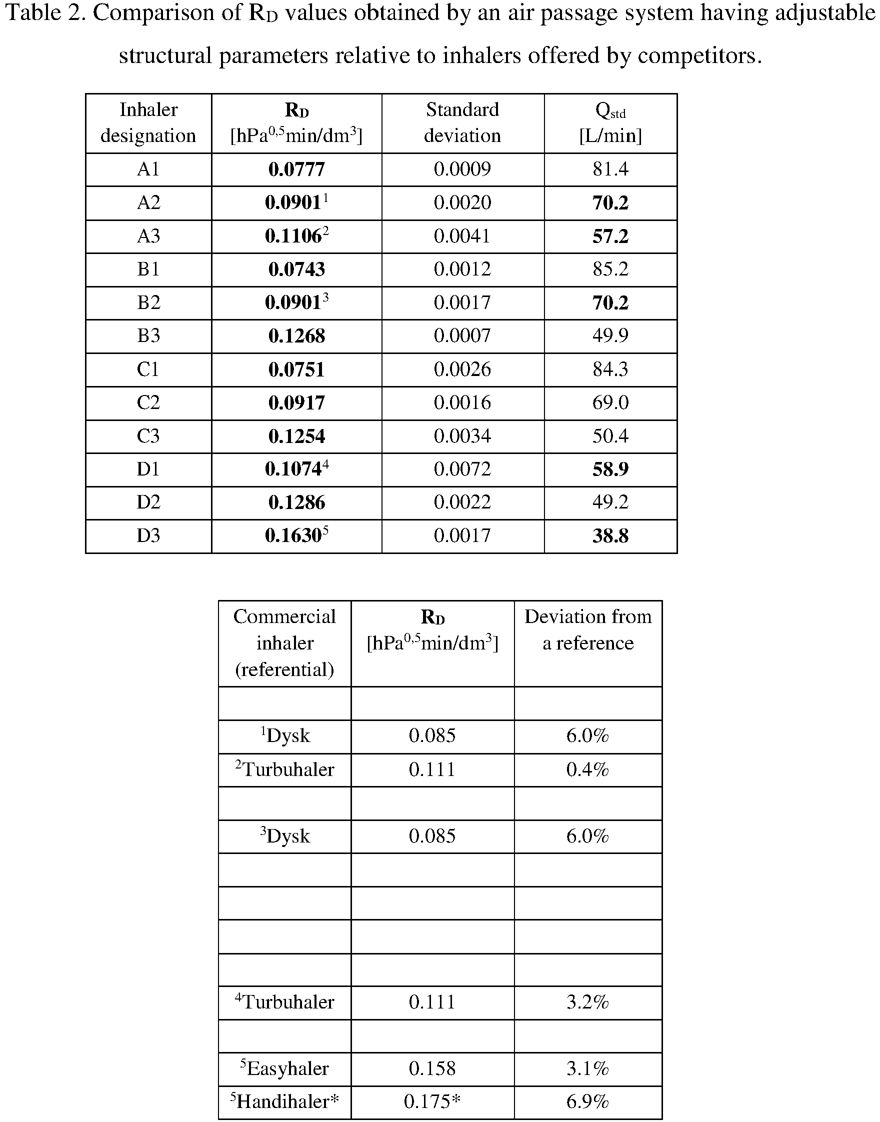

Owing to its modularity and application of different structural elements, the described solution allows selecting a suitable treatment and a medication having suitable parameters, which, on one hand, by means of suitable structural solutions, will be subjected to "de-aggregation," that is, detachment from the carrier particles, and, on the other hand, will be broken down into smaller fractions of a medication.

A "de-aggregation" process takes place already in a capsule following its perforation by means of the spikes 10 in the capsule chamber 14 in the glass 11. Holding the mouthpiece 21C in their mouth while inhaling, a patient sucks in the air which flows into the inhaler through a system of at least two air intake passages 25, which, in the described solution, are characterized by having a fixed height "H" and being capable of adjusting a suitable width "W" parameter. Application of a suitable ratio of the height parameter to the width has an influence on the characteristics of self-resistance of the device, as shown by the data (Table 1). The air flowing in through the passages 25 generates negative pressure which extracts a capsule from the capsule chamber 14 situated in the glass 11. The capsule enters the rotation chamber 26 in the mouthpiece base 21B, wherein it collides rotatingly with the chamber walls, as a result of which, the powder released from the capsule smashes against the chamber walls and the other particles rotating in the chamber. Subsequently, the aerosol being formed flows into the mouthpiece tube 21C through the mesh 21A located in the mouthpiece base 21B, separating the space of the rotation chamber 26 from the mouthpiece tube 21C, wherein the particles are further broken down by the poles of the mesh 21A. As a result of the rotational motion, the particles floating within the circular diameter of the tube channel are subjected to further fractioning by colliding with each other, and, when passing through the internal constriction 27, they are broken down further as a result of increased speed of the aerosol and their increased rotation following passing through the internal constriction 27. The results of the measurements in Table 1 - a significant change in the R_{D} values is possible in the case of a significant narrowing of the channel in the mouthpiece tube 21C. The final effect of the inhalation process is a release of an aerosol containing a medication through the mouthpiece tube 21C, through the mouth and into the upper airways and, depending on the content of fractions smaller than 10 µm, to the furthermost parts of the lower airways.

A comparison of the emitted aerosol particle size distribution in the few selected inhaler modules presented herein with the original Fantasmino inhaler (the dispersion was applied to Flutixon, measurements were performed using the diffraction method in a Spraytec spectrometer). Q_{std} airflows were applied corresponding (with the accuracy of ±5%) to the standard pressure decrease 4 kPA in each of the tested inhaler modules.

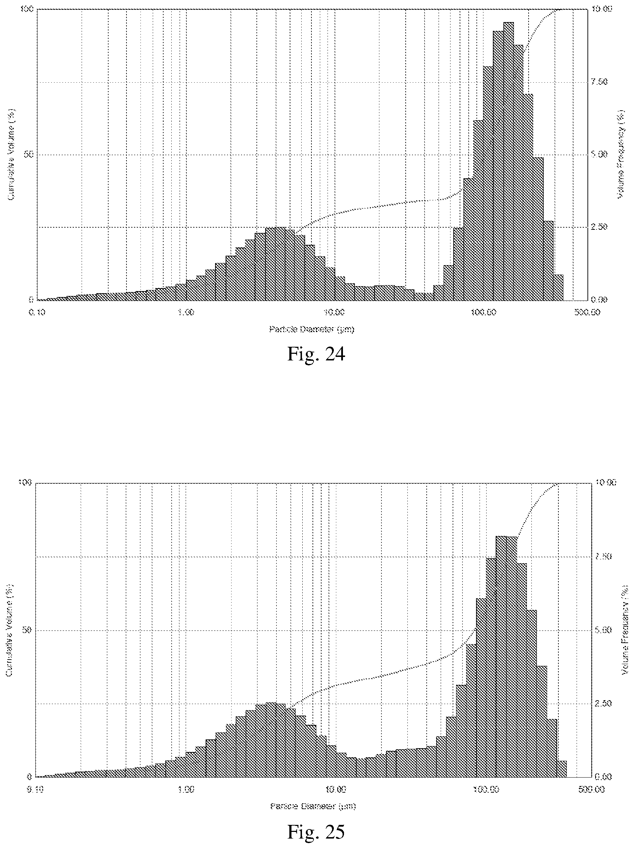

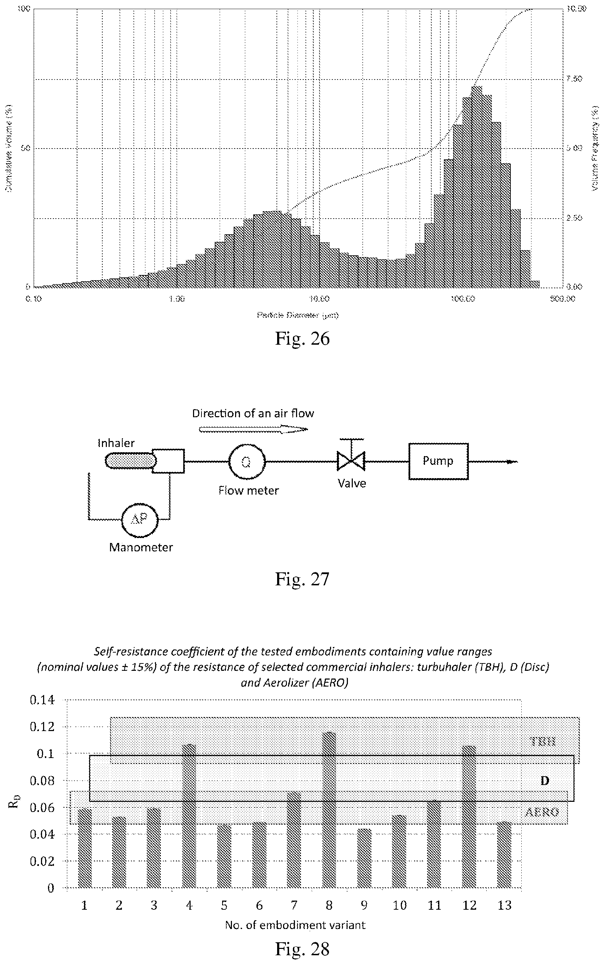

The results obtained for the D1 modules - 38% (Fig. 23), D3 - 29.5 % (Fig. 24), A2 - 31.2 % (Fig. 25) as compared with 34.5 % for the Fantasmino inhaler (Fig. 26), show that there are no differences in terms of the process of de-aggregation of a medicinal powder. An analysis of the proportion of small particles in a fraction of particles smaller than 10 µm has confirmed that in the case of the presented modules, de-aggregation was similar or even better than in the case of Fantasmino, and reached the following values: D1 - 69 %, D3 - 65 %, A1 - 72 %, Fantasmino - 65 % (particles < 4,7 µm). The results confirm the validity of application of an air passage system with adapted structural parameters possible as a result of application of modularity of the mouthpiece 21. The modularity is attained by using a suitable base 21B having a specific ratio of height "H" to width "W," mounting it by means of a latch 24, which is joined to the cover 15 and allows replacement of the entire module 21. Fitted into the base 21B so mounted is a mesh 21A and a mouthpiece tube 21C, which is joined to latch spots in the base by means of special latches 29. A cover 22 protecting the mouthpiece against pollution is placed on the mouthpiece tube 21C.

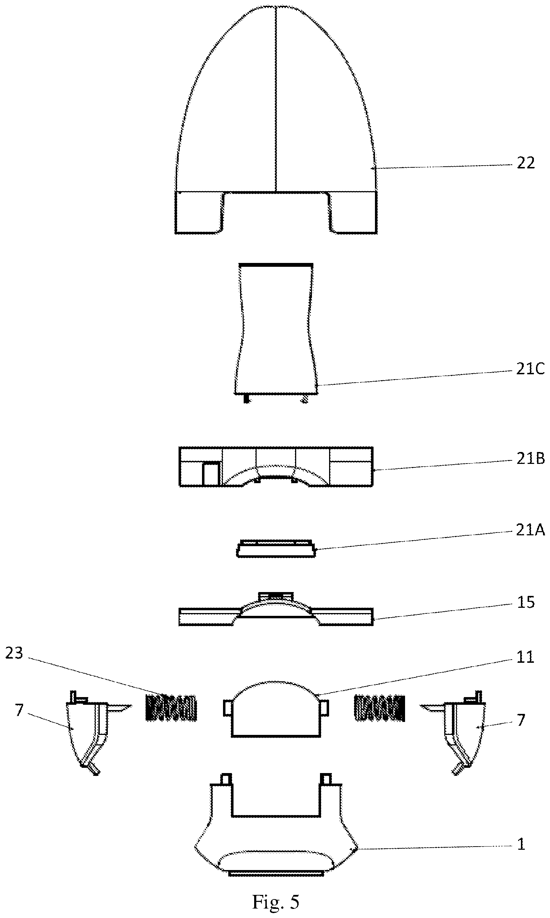

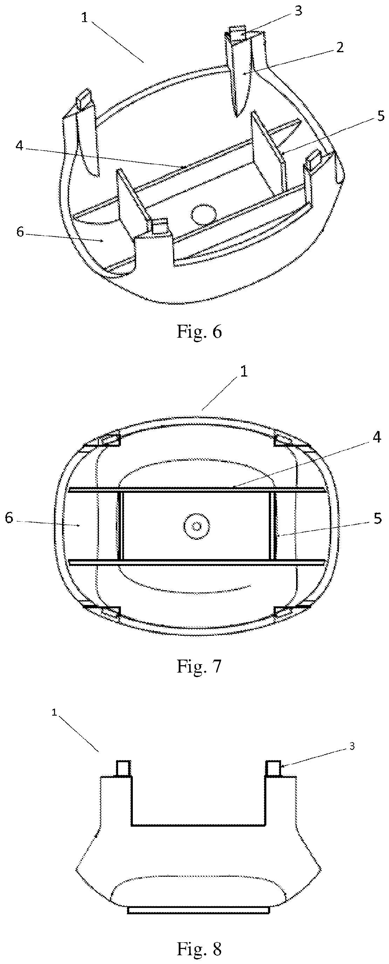

In other words, with regard to this, what is described above, a single-dose dry powder inhaler is shown generally in Fig. 5. The inhaler according to the present invention comprises a body 1 comprising four supporting pillars 2 arranged near the edge of said body substantially on a rectangular plan, as shown in Fig. 7, and protruding in a direction opposite to the bottom of said body 1. The ends of the supporting pillars 4 comprise latches 3. The body 1 of the inhaler comprises a body ribs system arranged within it. The body ribs system comprises two pairs of substantially parallel ribs, wherein one pair of the substantially parallel ribs 4 is substantially perpendicular to the other pair of the substantially parallel ribs 5, as shown in Fig. 6 and 7. Such an arrangement of the ribs 4, 5 forms a seat in the central part of the body 1, which contains a capsule chamber 14 adapted to receive a capsule with a medication. Further, the body of the inhaler comprises two guides 6 for guiding the push-buttons 7 for perforating the capsule. As shown in Fig. 6 and 7, the body 1 comprises two opposite guides 6 arranged on the sides of the body 1 and defined respectively on the sides by substantially parallel ribs 4 and ending with a rib 5.

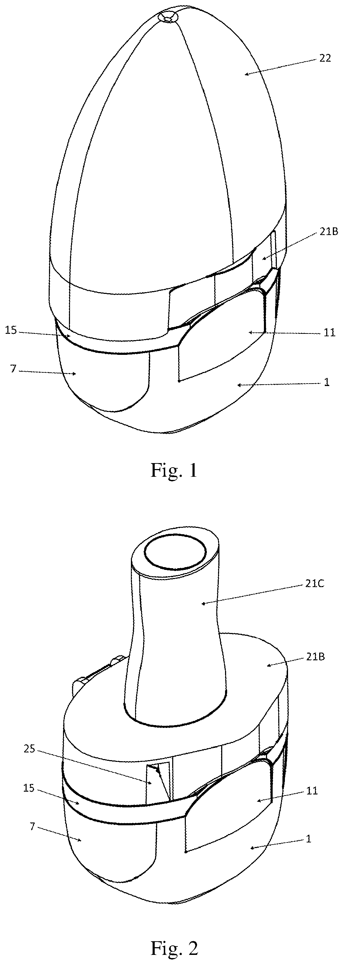

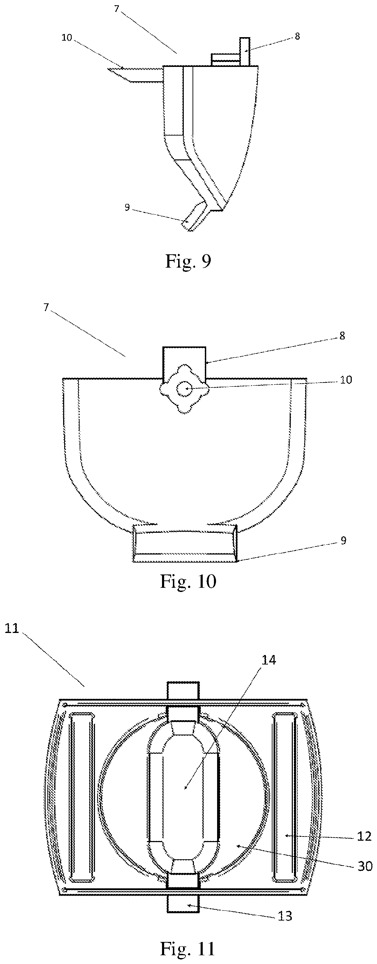

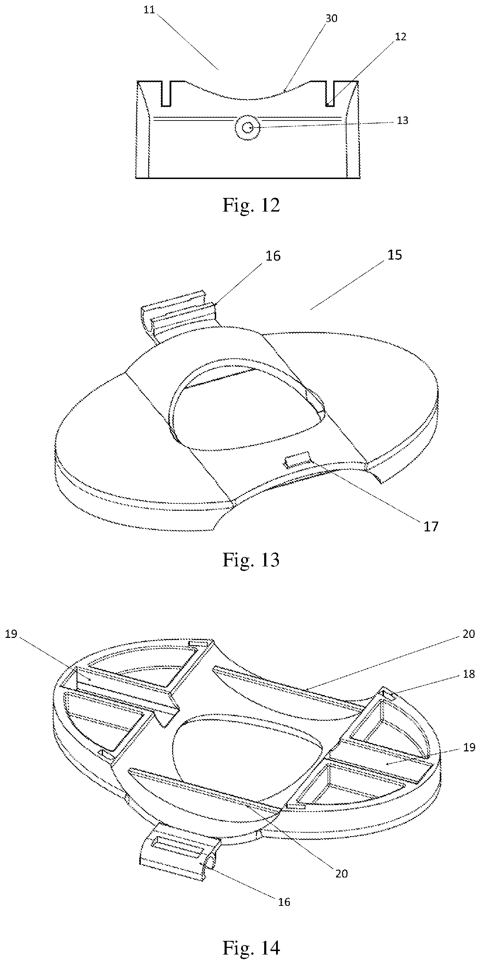

The inhaler according to the invention comprises a chamber element 11. The chamber element 11 comprises a substantially flat deck on one side of which there is a capsule chamber 14 in communication with an opening running through the deck of the chamber element 11 such as to allow access to the chamber 14 in order to place therein a capsule containing a medication. Furthermore, the chamber element 11 comprises side walls protruding from the deck of the chamber element 11 and surrounding the capsule chamber 14, as shown in Fig. 11 and 12. The capsule chamber 14 protrudes from the chamber element and has an elongated shape, as shown in Fig. 11. The capsule chamber 14 is slightly bigger than the capsule which is intended to be placed in said chamber 14. On the opposite sides of the capsule chamber 14 there are guide holes 13 for guiding the spikes 10 of the push-buttons. The guide holes 13 are arranged on the opposite sides of the capsule chamber 14 along the longitudinal axis of said chamber 14, as shown in Fig. 11, and run through the side walls of the chamber element 11. The capsule chamber 14 and side the walls of the chamber element 11 are made of a transparent material to provide visual access to the capsule chamber 14. The transparency of the walls of the chamber element 11 and the capsule chamber 14 are not essential for the invention. In another embodiment of the inhaler, the side walls of the chamber element 11 and/or the capsule chamber 14 are non-transparent. The opening of the capsule chamber 14 opens into the cavity 13 arranged on the deck of the chamber element 11 opposite the capsule chamber, as shown in Fig. 11 and 12. The chamber element 11 comprises also two substantially parallel, elongated recesses 12 arranged on the sides of the cavity 30 around the access opening of capsule chamber 14, as shown in Fig. 11 and 12. Said recesses 12 are used for coupling with the upper cover 15. As shown in Fig. 1 to 4, particularly in Fig. 5, the chamber element 11 is arranged in the body 1 of the inhaler such that the capsule chamber 14 is located in the seat of the body 1 between the ribs 4 and 5, wherein two of the opposite walls of the chamber element 11 are located between the pillars 3 in the front and in the back of the body 1, while the guide holes 13 on the two other side walls face the guides 6 of the body 1.

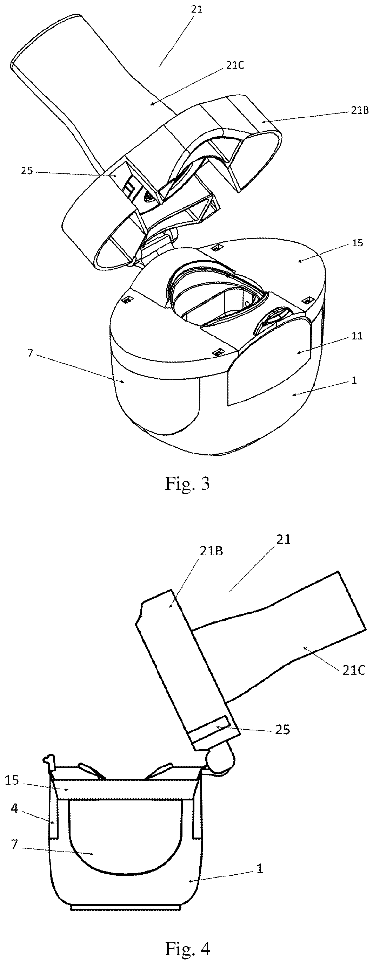

The inhaler according to the present invention comprises an upper cover 15 for closing the body 1 of the inhaler. The upper cover 15 comprises, substantially in its centre, a capsule chamber 14 access opening, as shown in Fig. 13. The size and alignment of the chamber 14 access opening of the upper cover 15 correspond to the cavity 13 in the chamber element 11, as shown in Fig. 3. On the underside of the upper cover 15 there are substantially parallel, elongated protrusions 20 extending on the opposite sides of the capsule chamber 14 access opening, as shown in Fig. 14. The shape and the arrangement of the protrusions 20 correspond with the shape and the arrangement of the recesses 12 of the chamber element 11. The upper cover 15 comprises also guides 19 for guiding the push-button 7 during perforation of a capsule in the capsule chamber 14. The upper cover 15 comprises two elongated guides 19 arranged opposite each other on the sides of said cover 15, as shown in Fig. 14. Furthermore, the upper cover comprises latch spots 18 of the upper cover 15. As shown in Fig. 14, the upper cover 15 comprises latch spots 18 in the form of four openings arranged such that they correspond with the latches 3 on the pillars 2 of the body 1. The upper cover 15 comprises a hinge groove 16 arranged transversely along the side of the cover 15, as shown in Fig. 14. The hinge groove 16 is an element for the formation of a hinge connection with the mouthpiece base 21C of the inhaler. On the opposite side of the hinge groove 16 there is a latch 17 arranged on the upper surface of the upper cover 15, as shown in Fig. 13. As shown in Fig. 1 to 4, the upper cover is placed on the body 1 such that the chamber element is immobilized between the body and the upper cover 15. The upper cover 15 is placed on the chamber element 11 and coupled with the body 1 by means of coupling the latches 3 with the openings in the latch spots 18 of the upper cover 15. The protrusions 20 of the upper cover 15 are placed in the recesses 12 of the chamber element 11 for better immobilization of the chamber element 11 in the body 1 and for defining the chamber space located above the cavity 30 of the chamber element 11 within the perimeter of the upper chamber 15. The guides 6 of the body 1 and the guides 19 of the upper cover are arranged opposite each other, forming two pairs of an upper guide and a lower guide, respectively, for each of the push-buttons 7.

The inhaler according to the present invention comprises push-buttons 7 for perforating a capsule in the capsule chamber 14. As shown in Fig. 9 and 10, the push-button 7 comprises a rounded plane with an edge, by means of which the push-button 7 rests on the body 1 of the inhaler. Near said edge and on the opposite sides of the internal side of the push-button 7, there is a lower protrusion 9 and an upper protrusion 8, respectively, as shown in Fig. 9 and 10. The lower protrusion of the push-button 7 is configured to move in the guide 6 of the body 1, while the upper protrusion 8 is configured to move in the guide 19 of the upper cover 15. Furthermore, the push-button 7 comprises a spike 10 for perforating a capsule in the capsule chamber 14, wherein the spike protrudes from inside the push-button 7 near the upper protrusion, as shown in Fig. 9 and 10. The push-button 7 comprises also a spring 23 for pushing the push-button 7 back following perforating a capsule in the capsule chamber 14. The inhaler according to the present invention comprises two push-buttons 7 arranged on the opposite sides of the body 1, as shown in Fig. 1 to 4, particularly in Fig. 5. Each of the push-buttons 7 is arranged in the body 1 of the inhaler such that a push-button 7 rests by means of its edge on the inside of the body 1. The upper protrusion 8 of the push-button 7 is arranged in the guide 19 of the upper cover 15, while the lower protrusion 9 of the push-button is arranged in the guide 6 of the body. The spike 10 is arranged in the guide hole 13 extending to the capsule chamber. Between the push-button 7 and the side of the capsule chamber 14, near the guide hole 13, there is a loaded spring 23 arranged such that the push-button 7 is continuously pushed away from the capsule chamber 14 and rests by means of its edge on the body 1.

Thus, as described above, the body 1, the chamber element 11, the upper cover 15 and the push-button 7 form together the body assembly. The inhaler body assembly according to the present invention provides a chamber 14 for placing therein a capsule containing a medication, in which chamber 14 the capsule is perforated during the operation of the inhaler. Furthermore, the inhaler body assembly comprises push-buttons 7 with spikes 10, thus providing means for perforating a capsule containing a medication.

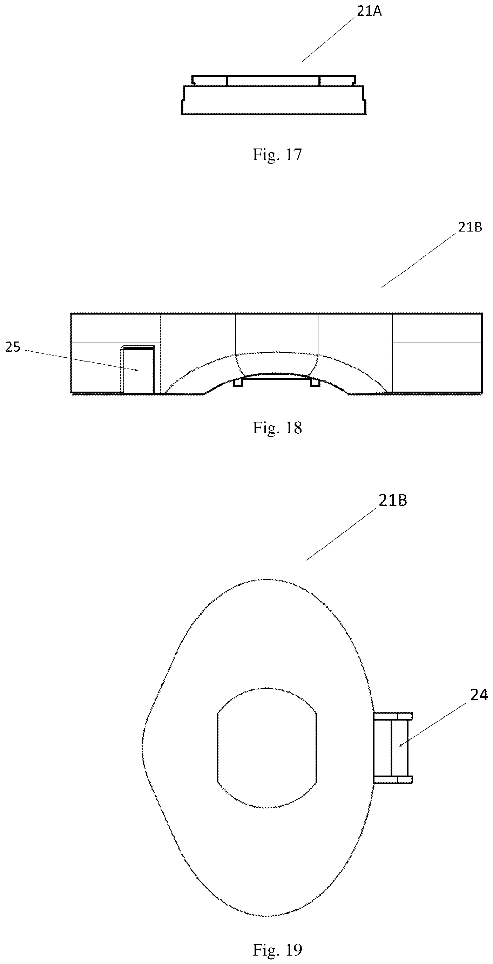

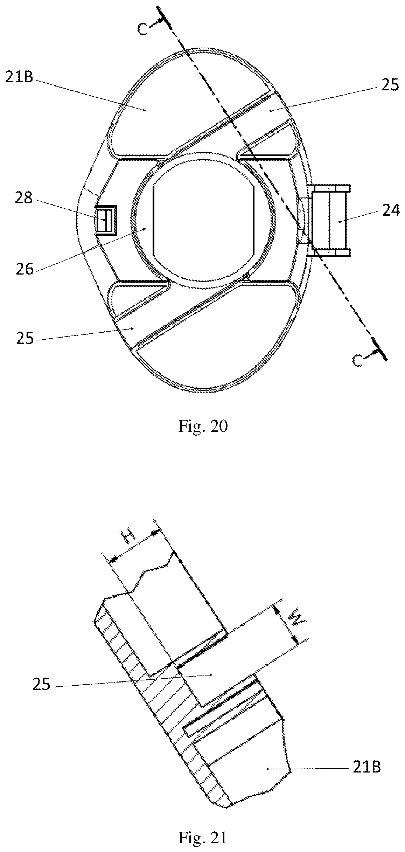

The inhaler according to the present invention comprises a mouthpiece base 21B. The mouthpiece base 21B comprises a rotation chamber 26 with an opening running to the tube 21C. As shown in Fig. 19, the opening of the rotation chamber 26 is substantially arranged in the centre of the mouthpiece base 21B. On the lower side of the base 21B, the size and the arrangement of the rotation chamber 26 correspond with the opening in the upper cover 15 of the body 1 of the inhaler, as a result of which, during the operation of the inhaler, the rotation chamber 26 is in flow communication with the capsule chamber 14. The rotation chamber 26 of the base 21B is used for extracting a medication from a perforated capsule and for its de-aggregation. The mouthpiece base 21B comprises air intake passages 25 for supplying air into the inhaler during its application for inhaling a medication. As shown in Fig. 20, the mouthpiece base 21B comprises two air intake passages 25 arranged on its opposite sides and tangentially to the rotation chamber 26. The two air intake passages 25 comprise substantially straight passages running from the opposite sides of the mouthpiece base 21 B and substantially parallel to each other, respectively, as shown in Fig. 20. The air intake passage 25 has substantially a quadrilateral cross-section, as shown in Fig. 21, and is described by means of parameters constituting its height "H" and width "W." As described above, changing at least one of the height "H" and width "W" of the air intake passages 25 such that the ratio of the height "H" to the width "W" is changed, causes a change in the characteristics of self-resistance of the inhaler according to the invention, as described and shown in Table 1 above. The above-described system of air intake passages 25 is one of the possible systems of air intake passages. Different systems of air intake passages are also possible. For example, depending on the needs, one, two or more air intake passages can be applied. Moreover, arrangement of an air intake passage/air intake passages are adaptable depending on the needs and the number of the air intake passages applied. The geometry of the cross-section of the air intake passages can also differ from that described above. Namely, air intake passages can be used, having a cross section selected from a circular, oval, square, etc. cross-sections. Irrespective of the shape of an air intake passage, the essence of the invention is a change in at least one of the geometrical parameters of an air intake passage, resulting in a change in the cross-section of such a passage, also referred as the inside measurement of the passage, which results in a change in the characteristics of self-resistance of the inhaler, as described above. As already described above, by providing a mouthpiece base 21B having a specific ratio of height "H" to width "W" of the air intake passages, an inhaler is provided having a specific self-resistance coefficient, and by providing a plurality of mouthpiece bases 21B having a specific ratio of height "H" to width "W" of the air intake passages, self-resistance of the inhaler according to the present invention is adjustable for a specific medication. In a preferred embodiment, a plurality of the mouthpiece bases 21B is provided, comprising air intake passages 25 having the same height "H" and different widths "W," as described above in detail. The ratio of one of the dimensions of the air intake passage 25 to the other dimension of the air intake passage is in the range from 1:1 to 1:0.10, preferably from 1:0.08 do 1:0.25. In one of the preferred embodiments, the ratio of the height "H" of the air intake passage 25 to the width "W" of the air intake passage is in the range from 1:1 to 1:0.10, preferably from 1:0.08 do 1:0.25. In another preferred embodiment, the ratio of the width "W" of the air intake passage 25 to the height "H" of the air intake passage is in the range from 1:1 to 1:0.10, preferably from 1:0.08 do 1:0.25. In the case of other geometries of the air intake passage, the height "H" and the width "W" correspond to the long dimension and the short dimension, respectively, of the geometrical shape of the cross-section of such a passage 25. The mouthpiece base 21B also comprises a hinge grip 24 on the side of the mouthpiece base 21B, as shown in Fig. 20, wherein said grip 24 is arranged complementary with the hinge groove 16 of the upper cover 15 to form a hinge connection, as shown in Fig. 2 and 3. Moreover, the mouthpiece base 21B comprises a latch spot 25 of the mouthpiece base 21B. As shown in Fig. 20, the mouthpiece base 21B comprises a latch spot 25 in the form of an opening on the underside of the mouthpiece base 21B near the rotation chamber 26 and opposite the hinge grip 24, wherein the opening of the latch spot 25 is arranged such that it corresponds with the latch 17 of the upper cover 15 of the body assembly.

The inhaler according to the invention comprises a mouthpiece mesh 21A. The mouthpiece mesh 21A comprises poles on which a medication is de-aggregated, as described above. In addition, the mouthpiece mesh 21A prevents too large particles of a not de-aggregated medication and capsule coating particles from entering a patient's airways. The mouthpiece mesh 21A is arranged in the opening of the rotation chamber 26 of the mouthpiece base 21B, as shown in Fig. 5. The mouthpiece mesh 21A comprises latch spots of the mouthpiece tube 21C, not shown in the figures. The mouthpiece mesh 21A comprises latch spots in the form of two notches arranged on the opposite sides of the edge of the mesh 21A.

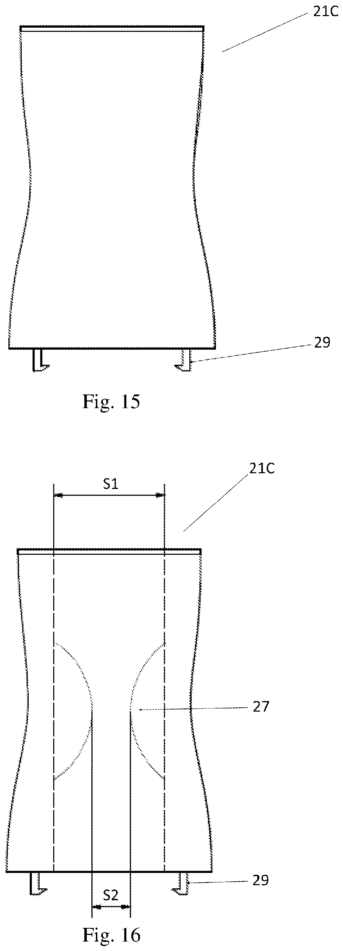

The inhaler according to the invention comprises a mouthpiece tube 21C. The tube 21C constitutes a pipe element one end of which is configured to be placed in a patient's mouth for inhaling a medication from an inhaler according to the invention. The other side of the tube 21C comprises latches for coupling with the mesh 21A. Inside the tube 21C there is a tube constriction 27, which decreases the through diameter of the tube 21C for increasing the de-aggregation of a medication by an inhaler according to the invention. The size of the through diameter of the mouthpiece tube 21C is adjustable by means of the constriction 27 depending on the needs, thus regulating the clearance of the tube. The size of the constriction 27 of the mouthpiece tube 21C can be freely adjusted such that the percentage of coverage of the internal passage of the mouthpiece tube 21C is the range of 0% - 100%. The percentage of coverage of the passage of the mouthpiece tube 21C is practically adjustable within a range of 0% - 95%. As already mentioned, the percentage of coverage of the passage of the mouthpiece tube 21C is adjustable within a range of 0%-85%. For the percentage of coverage equalling 0%, the constriction 27 of the passage of the mouthpiece tube 21C equals zero. In other words, for the clearance of the passage of the mouthpiece tube 21C equalling 0% of coverage, the internal passage of the mouthpiece tube 21C does not contain a constriction. By changing the size of the constriction 27 of the tube 21C and, consequently, the percentage of coverage of the internal passage of the mouthpiece tube 21C, it is possible to adjust de-aggregation of a medication, thus allowing adapting an inhaler according to the present invention to the needs of a patient undergoing a treatment involving at least one medication. As already described above, by providing a mouthpiece tube 21C having a specific constriction 27, an inhaler is provided providing particles of an active substance of a medication smaller than 10 µm for a specific medication, and by providing a plurality of mouthpiece tubes 21C having a specific internal diameter, an inhaler according to the present invention can be adjusted for specific medications ensuring de-aggregation of particles of an active substance of a medication smaller than 10 µm. The mouthpiece tube 21C comprises latches 29 for joining with the mesh 21A. As shown in Fig. 15 and 16, the mouthpiece tube comprises two latches 29 arranged at the end of the tube, which is located opposite the end adapted to be placed in a patient's mouth. The latches 29 of the mouthpiece tube 21C are arranged such that they correspond with the latch sites of the mouthpiece mesh 21A.

The mesh 21A is placed in the opening of the rotation chamber 26 of the mouthpiece base 21B, with which mesh 21A the mouthpiece tube 21C is joined by means of the tube latches 29 and the mesh latch sites to create the mouthpiece 21 of the inhaler of the present invention.

Thus, as described above, the mouthpiece base 21B, the mouthpiece mesh 21A and the mouthpiece tube 21C form together the mouthpiece assembly. The mouthpiece assembly of the inhaler according to the invention provides intake passages 25 for supplying air into the inhaler, a rotation chamber 25 for releasing a medication from a capsule, de-aggregating it and forming an aerosol with the air supplied through the intake passages 25, as well as the tube 21B for further de-aggregation of an aerosolized medication and for its inhaling by a patient through the mouthpiece 21.

The mouthpiece assembly described above is a modular mouthpiece. This means that a series of mouthpiece bases 21B is produced, having different geometries of air intake passages 25, constituting a generic array with increasingly smaller inside measurement of the air intake passages 25, as described above. In other words, a series of mouthpiece bases 21B are produced, having equal ratios of the width "W" of the air intake passages 25 to their height "H" with an increasingly smaller inside measurement of the air intake passages 25. The particular mouthpiece bases 21B in an array constitute modules of the mouthpiece base. Separately, a series of tubes 21C are produced, having different constriction 27 sizes, constituting a generic array with increasingly smaller through diameters. Particular mouthpiece tubes 21C in an array constitute modules of the mouthpiece tube. To adjust the working parameters of the inhaler according to the invention to a specific medication, a suitable mouthpiece base module and a suitable mouthpiece tube module are selected and assembled together as described above, to arrive at a suitable mouthpiece assembly to be fitted in the inhaler, as described above.

In another embodiment of the invention, the mouthpiece base 21B, the mouthpiece mesh 21A and the mouthpiece tube 21C, forming together the mouthpiece assembly, are mutually integrated. In such case, a plurality of generic arrays for different selected geometries of the air intake passages 25 are produced. This means that a generic array of mouthpieces 21 are produced, wherein the inside measurement of the air intake passages 25 in the mouthpiece base 21C is fixed, and the size of the constriction 27 of the mouthpiece tube 21C changes in the array. Next, another generic array is produced, where the inside measurement of the air intake passages 25 in the mouthpiece base 21C is fixed but has a value different from that in the previous array, while in the current array the constriction 27 of the mouthpiece tube 21C changes. Such arrays of the mouthpieces 21 are produced for all required inside measurements of intake passages 25 of different sizes of constriction 27 of the mouthpiece tube 21C. In other words, series of mouthpieces 21 are produced in generic arrays for the required values of the ratios of the widths "W" of the air intake passages 25 to their heights "H" and for the required sizes of the constriction 27 in the mouthpiece tube 21C. In such a case, in order to adjust the working parameters of the inhaler according to the invention to a specific medication, a suitable mouthpiece 21 is selected as an integrated module and mounted in the inhaler as a mouthpiece assembly, as described above.

The body assembly is removably hinge-connected to the mouthpiece assembly such that the mouthpiece assembly can be tilted away from the body assembly from a closed position to an open position. The hinge groove 16 of the upper cover 16 is latched on the hinge grip 24 of the mouthpiece base 21B to form a fastening connection, a hinge connection, allowing the mouthpiece base 21C to tilt away rotatingly relative to the upper cover 15, as shown in Fig. 2 to 4. This way the inhaler can be closed and opened, *i.e.,* transformed from a closed configuration to an open configuration, and the other way around. In the closed configuration of the inhaler, *i.e.,* when the inhaler is closed, the mouthpiece base 21B rests on the upper cover 15, as shown in Fig. 2, and they are interconnected by means of the latch 17 of the upper cover 15 latched in the opening of the latch space 28 in the mouthpiece base 21C. In the closed configuration, the rotating chamber 26 is joined to the capsule chamber 14. After laterally pressing the mouthpiece assembly, the latch 17 of the upper cover 15 is disconnected from the opening of the latch space 28 in the mouthpiece base 21C and the upper cover 15 rotates relative to the base 21C by means of a rotation of the hinge grip 24 of the mouthpiece base 21C in the hinge groove 16 of the upper cover 15 and the inhaler is transformed into the open configuration. In the open configuration of the inhaler, *i.e.,* when the inhaler is open, the mouthpiece base 21B is tilted away by means of the hinges from the upper cover 15, as shown in Fig. 3. In the open configuration, the rotation chamber 26 of the inhaler is disconnected from the capsule chamber 14 to provide access to the capsule chamber 14 in order to remove potential residues of a capsule following inhalation and/or in order to introduce a new inhalation capsule. Other methods of mounting the mouthpiece assembly on the body assembly can also be used such as to provide opening and closing of the inhaler according to the invention. For example, the upper cover 15 and the mouthpiece base 21B can comprise connecting means allowing the mouthpiece base 21B to slide over the upper cover 15 and their secure locking relative to each other in a similar manner, as described above for the closed configuration, and allowing the mouthpiece base to slide off of the upper cover 15 to provide access to the capsule chamber 14 in a similar manner, as described above for the open configuration. Closing the inhaler, *i.e.,* transforming it into the closed configuration, is effected in a similar manner as described above, except in a reverse order.

The inhaler of the invention comprises a cover 22 placed over the mouthpiece tube 21C and a mouthpiece base 21, as shown in Fig. 1, to secure the mouthpiece 21 when the inhaler is not in use. To use the inhaler of the invention, the cover 22 is removed to uncover the mouthpiece assembly. After the inhaler according to the invention is no longer used, the cover 22 is put back in the position as described above.

Now, general operation of the inhaler according to the invention will be described, whereby its operation is the same for any geometry of the air intake passages 25 in the mouthpiece base 21B and/or the constriction 27 in the mouthpiece tube 21C. The cover 22, if being in place, is removed from the inhaler and is opened as described above, if closed. Next, a capsule containing a medication is placed in the capsule chamber 14, and the inhaler according to the invention is closed, as described above. The push-buttons 7 are pressed resulting in the spikes 10 of the corresponding push-button 7 moving in the guide holes 13 to perforate the capsule containing a medication in the capsule chamber 14. After releasing the pressure exerted on the push-buttons 7, the springs 23 cause them to return to their initial position and cause the spikes 10 to retreat from the capsule chamber 14. Next, air is sucked in through the free end of the mouthpiece tube 21C such that negative pressure is generated in the inhaler causing air to be sucked into the inhaler through the air intake passages 25 in the mouthpiece base 21B. Air is inhaled by a patient through the mouthpiece 21 to inhale a medication from a capsule or by a testing device for testing the properties of the inhaler according to the invention. The negative pressure generated in the inhaler according to the invention causes a perforated capsule to be entrained from the capsule chamber 15 of the chamber element 11 to the rotation chamber 26 of the mouthpiece base 21B. Simultaneously, the air sucked into the inhaler according to the invention by the air intake passages 25 of the mouthpiece base is caused to rotate intensively in the rotation chamber 26. The intensive rotation of air causes the capsule to collide rotatingly against the walls of the rotation chamber 26 to release therefrom a medication and to cause its de-aggregation as a result of the particles of the medication colliding against each other, against the capsule remains and against the rotation chamber walls 26 in the rotating stream of air, resulting in a decrease in the size of the particles of the medication. This causes the formation of an aerosol of particles of the medication suspended in the air sucked into the inhaler through the air intake passages 26. Next, the aerosol passes into the mouthpiece tube 21C through the mouthpiece mesh 21A, wherein its poles cause potential further de-aggregation of the medication in aerosol and, consequently, a reduction in the size of the particles of the medication. While passing through the mouthpiece tube 21C with the constriction 27, the aerosol is subjected therein to a strong flow disturbance. As a result of said disturbance, the particles of the medication collide multiple times against each other and against the walls inside the tube 21C, in particular, against the constriction 27 in the way of the flow, if any, causing an even bigger de-aggregation of the particles of the medication, hence the size of its particles suspended in the air. After passing through the constriction 27, the aerosol of the medication leaves the mouthpiece tube 21C of the inhaler according to the invention. As a patient inhales air through the inhaler, the aerosol of the medication enters the patient's airways. In the case when air is inhaled through the inhaler by a testing device, the aerosol inhaled through the tube 21C is subjected to testing to determine the characteristics of the inhaler with the preset geometries of the components of the inhaler according to the invention and to determine the distribution of the particle size of the medication tested. Next, the inhaler of the invention is open, as described above, and the inhalation residues, if any, are removed from the capsule chamber 14 and the rotation chamber 26. The inhaler is then ready for another inhalation, as descried above. If the inhaler is no longer to be used, it is closed, as described above, and the cover 22 is placed on the mouthpiece assembly.

The Inventors have tested the inhaler according to the invention to determine self-resistance of the inhaler while providing an aerosol with active substance particles smaller than 10 µm.

The tests of the inhaler according to the invention in the first series for the first version models are described in detail above. These models were made of resin by means of 3D printing. To summarize the above detailed description, a series of modules 1, 2, 3 of mouthpiece bases 21C is provided having a ratio of the width "W" to the height "H" of 1:0.85, 1:0.50 and 1:0.25, respectively, and a series of A, B, C, D mouthpiece tubes 21B having the sizes of the constriction 27 resulting in an internal diameter of 0% of coverage (no constriction), 33% of coverage, 66% of coverage and 85% of coverage, respectively, as shown in Table 1 above. The inhaler has been tested in terms of the R_{D} coefficient values for the particular modules for all combinations of the base modules 21 B and the tube modules 21C, and the results are shown in Table 1 above. Subsequently, as described above, selected combinations of the mouthpiece base 21B and the mouthpiece tube 21C were tested for de-aggregation of the medication tested. The test results for particular combinations of the mouthpiece base 21B and the mouthpiece tube 21C of the inhaler according to the invention are presented in the graphs of Fig. 23 to 26, as discussed above in detail. On the above-mentioned graphs, two maximums for specific combinations of the mouthpiece base 21B and the mouthpiece tube 21C are observed in the particle size distribution, namely, one below 10 µm and one above 30 µm, with a minimum of 10 to 30 µm therebetween. The particle size distribution below 10 µm describes the particle distribution of a medication of a fraction containing mostly the active substance and, as the particle size increases, the amount of the active substance increases. The particle size distribution above 30 µm describes the particle distribution of a medication containing mostly a carrier and, as the particle size increases, the amount of the carrier increases. A qualitative fraction analysis for the particular particle sizes shows that a particle fraction of a medication falling within a maximum of below 10 µm and less contains practically only active substance particles of a medication, while a particle fraction of a medication falling within a maximum below 20 µm and more contains only carrier particles of a medication, while the range of 10-30 µm contains the particles of both the active substance and the carrier of the tested medication. The medication tested contained Flutixon as the active substance and lactose as the carrier.

The Inventors have tested the inhaler of the invention also in the second series for the first and the second version for different combinations of the geometries of the components of the inhaler according to the invention in terms of its self-resistance. The test models in the second version were made of resin using a 3D printer, wherein one of them was made of ABS using injection technology.

The inhalers of the invention have been tested using the measurement system shown in Fig. 27. The inhalers' resistance was measured using the standard method, by means of which a pressure drop in the inhaler *ΔP* was measured using a digital manometer by TESTO, Germany, in the function of an air volume stream Q measured using an anemometric flow meter by TSI Inc., USA, sucked in by the inhaler by means of a high-efficiency vacuum pump by Vacuubrand, Germany.

The tests were carried out using integrated inhaler mouthpieces 21 having the geometries specified in Tables 3 and 4 below for particular versions. The data shown in Table 3 constitutes source data for the first-version inhalers, based on which the data shown in Table 1 above was prepared.

**Table 3. Geometries of particular elements of the mouthpiece of the version 1 inhaler for testing the mouthpiece 21.**

| Air intake passage 25 in the mouthpiece base 21C (height "H," width "W") | Mouthpiece mesh 21A size | Constriction 27 inside the tube 21C (diameter, inside measurement) |
|---|---|---|
| *K01* (5.0 mm, 4.23 mm) | S01 (1x1 mm) | *A01* (11.0 mm, 11.0 mm)* |
| *K02* (5.0 mm, 2.54 mm) | | *A02* (11.0 mm, 8.8 mm) |
| *K03* (5.0 mm, 1.27 mm) | | *A03* (11.0 mm, 6.6 mm) |
| | | A04 (11.0 mm, 4.4 mm) |
| * the size of the bottleneck 27 of the mouthpiece tube 21C equals 0 | | |

**Table 4. Geometries of particular elements of the mouthpiece of the version 2 inhaler for testing the mouthpiece 21.**

| Air intake passage 25 in the mouthpiece base 21C (height "H," width "W") | Mouthpiece mesh 21A size | Constriction 27 inside the tube 21C (diameter, inside measurement) |
|---|---|---|
| K01 (8.5 mm, 4.23 mm) | S01 (1x1 mm) | A01 (11.0 mm, 11.0 mm)^{∗} |
| K02 (8.5 mm, 2.54 mm) | | A02 (11.0 mm, 8.8 mm |
| K03 (8.5 mm, 1.27 mm) | | A03 (11.0 mm, 6.6 mm) |
| | | A04 (11.0 mm, 4.4 mm) |
| * the size of the constriction 27 of the mouthpiece tube 21C equals 0 | | |

The inhaler of the invention was tested in terms of integrated mouthpieces 21 in the geometrical variants, as presented inT 5 below, wherein the geometrical features of the mouthpiece 21 are shown by means of the codes from Tables 3 and 4 above for the first and the second version of the inhaler, respectively.

**Table 5. Design variants of the models of the tested mouthpieces 21 of the inhalers according to the invention.**

| No. of the geometrical variant of the mouthpiece 21 | Geometrical features of the mouthpiece 21 |
|---|---|
| 1 | K01 - A01 |
| 2 | K01 - A02 |
| 3 | K01 - A03 |
| 4 | K01 - A04 |
| 5 | K02 - A01 |
| 6 | K02 - A02 |
| 7 | K02 - A03 |
| 8 | K02 - A04 |
| 9 | K03 - A01 |
| 10 | K03- -A02 |
| 11 | K03 - A03 |
| 12 | K03 - A04 |
| 13^{∗} | K01 - A01 |
| * A variant made of ABS and only for the second version | |

Inhalers comprising mouthpiece 21 variants have been tested by means of the above-described measurement system to determine self-resistance of the inhaler for the particular variants of the mouthpiece 21 in the second version. The aggregate results of the measurements for particular variants of the first and second versions are presented in Table 6 below.

**Table 6. Average values of self-resistance coefficients of inhalers with different geometrical variants of the mouthpiece 21.**

| No. of the geometrical variant of the mouthpiece 21 | **Average value of the internal resistance coefficients R_{D} [hPa^{0,5}min/L] Version 1** | **Average value of the internal resistance coefficients R_{D} [hPa^{0,5}min/L] Version 2** |
|---|---|---|
| 1 | **0.0695** | **0.0629** |
| 2 | **0.0705** | **0.0705** |
| 3 | **0.0782** | **0.0782** |
| 4 | **0.1332** | **0.1332** |
| 5 | **0.0775** | **0.0775** |
| 6 | **0.0890** | **0.0890** |
| 7 | **0.0902** | **0.0902** |
| 8 | **0.1691** | **0.1691** |
| 9 | **0.0919** | **0.0919** |
| 10 | **0.0981** | **0.0981** |
| 11 | **0.1146** | **0.1146** |
| 12 | **0.1512** | **0.1570** |
| 13 | **-** | **0.0491** |

Fig. 28 is a graphic representation of the results from Table 6 and only for the second version, where for comparison, the graph contains the ranges of the nominal values ±15% of the internal resistance of selected commercial inhalers Turbuhaler (TBH), D (Disc) and Aerolized (AERO). Moreover, Table 7 below shows the resistance of selected dry-powder inhalers, including the indication of the data source.

**Table 7. Resistance of selected commercial dry-powder inhalers.**

| | Nominal internal resistance R_{D} [hPa^{0,5}min/L] | Allowable range of internal resistance values according the EMA recommendations |
|---|---|---|
| Breezhaler | 0,054¹ | 0,046 - 0,062 |
| Aerolizer | 0,058² - 0,060¹ | 0,049 - 0,069 |
| Dysk | 0,076^{2,3} - 0,085¹ | 0,065 - 0,098 |
| Novolizer | 0,085^{1,2} | 0,072 - 0,098 |
| Genuair | 0,098¹ | 0,083 - 0,113 |
| Turbuhaler (Symbicort) | 0,111¹ | 0,094 - 0,128 |
| Turbuhaler (Pulmicort) | 0,123¹ - 0,130² | 0,105 - 0,150 |
| Easyhaler | 0,158¹ | 0,134 - 0,182 |
| Handihaler | 00,183^{1,4} | 0,156 - 0,955 |
| 1. Kruger P., Ehrlein B., Zier M., Greguletz R. (2014). Inspiratory flow resistance of marketed dry powder inhalers (DPI). Eur Respir J 44 Suppl 58, 4635. | | |
| 2. Sosnowski T.R., Gradoń L. 2004. Badanie oporów aerodynamicznych inhalatorów proszkowych. Inz. Chem. Proces. 25, 1619-1625. | | |
| 3. Hejduk A., Urbańska A., Osiński A., Lukaszewicz P., Domański M., Sosnowski T.R. (2018). Technical challenges in obtaining an optimized powder/DPI combination for inhalation delivery of a bi-component generic drug. J. Drug Deliv. Sci. Technol. 44, 406-414. | | |
| 4. Sosnowski T.R. (2017). Own research. | | |

The test results of an inhaler according to the invention comprising mouthpiece 21 variants show that it is operable within the entire range of self-resistance of inhalers. In other words, as explained above, such geometrical parameters of an inhaler according to the invention can be selected as to allow it to operate within a scope ranging from a low-resistance to a high-resistance inhaler. The test results, however, indicate that low and medium self-resistance of the inhaler are the easiest to achieve. This range offers the biggest selection of the methods of attaining this objective by narrowing down the air intake passages 25 in the mouthpiece base 21B and/or by decreasing the internal diameter of the mouthpiece tube 21C. A local constriction 27 of the mouthpiece tube 21C, however, contributes to a better de-aggregation of the particles emitted by the inhaler and, consequently, to arrive at a bigger amount of fractions smaller than 10 µm entering a patient's mouth.

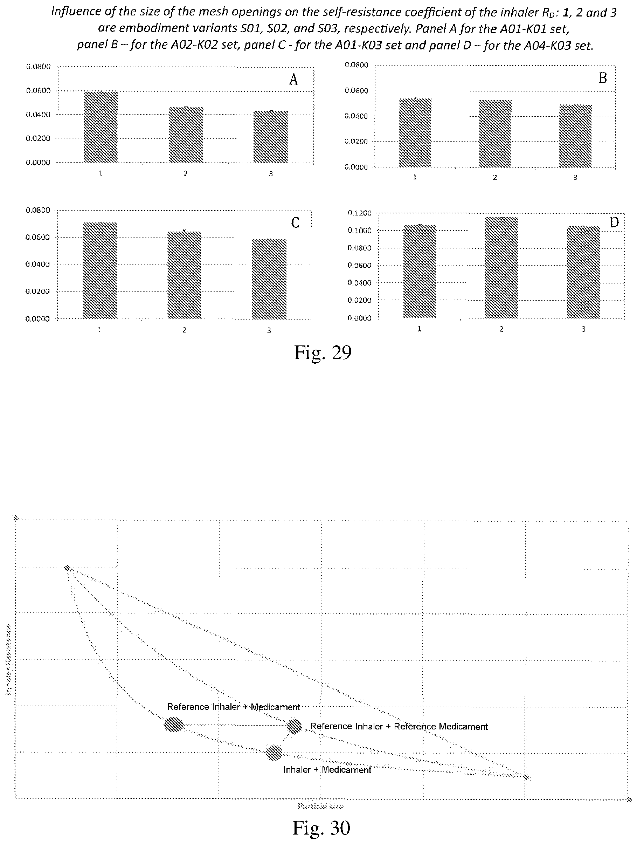

The Inventors have also analyzed the influence of the size of the openings of the mouthpiece mesh 21A on the self-resistance coefficient of the inhaler according to the invention, wherein the geometries of the components of the mouthpiece 21 were as presented in Table 8. Fig. 29 shows test results for inhalers according to the invention, comprising mouthpiece meshes 21A with different sizes of openings. As shown in Fig. 29, four series of mouthpieces have been tested having the following geometries, panel A - K01-A01, panel B - K02-A02, panel C - K03-A01 and panel D - K03-A04, respectively, for the mesh 21A S01, S02, S03. As shown, decreasing the mesh openings causes a certain decrease in the resistance, but only in the case of a simple or slightly constricted mouthpiece tube 21B. For a mouthpiece tube 21C having a large constriction A04, an increase in the size of the openings of the mouthpiece mesh 21A may decrease or increase self-resistance of the inhaler, as observed for S03 and S02, respectively.

**Table 8. Geometries of particular elements of the mouthpiece of the version 2 inhaler for testing the mouthpiece 21.**

| Air intake passage 25 in the mouthpiece base 21C | Mouthpiece mesh 21A size | Constriction 27 inside the tube 21C |
|---|---|---|
| (height "H," width "W") | | (diameter, inside measurement |
| K01 (8.5 mm, 4.23 mm) | S01 (1x1 mm) | A01 (11.0 mm, 11.0 mm) |
| K02 (8.5 mm, 2.54 mm) | S02 (2×1 mm) | A02 (11.0 mm, 8.8 mm |
| K03 (8.5 mm, 1.27 mm) | S03 (1.5×1.5 mm) | A04 (11.0 mm, 4.4 mm) |

As described above, the test results in the first series confirm that the proposed inhaler according to the invention, having variable geometry of the mouthpiece 21 is characterized by a wide range of internal resistance allowing to generate the resistance of typical commercial dry-powder inhalers. Increased internal resistance can be generated by providing air intake passages 25 of a mouthpiece base 21B having a decreased cross-section and a mouthpiece tube 21C having a big constriction 27.

Thus, the present invention discloses a universal single-dose dry powder inhaler, which, depending on the geometry of the components of the mouthpiece 21 can be used within a broad range of the self-resistance coefficient, also referred to herein as specific resistance, internal resistance, resistivity or simply resistance of the inhaler, from high-resistance inhalers to medium-resistance inhalers to low-resistance inhalers. Furthermore, regardless of the internal resistance of a given inhaler, it ensures de-aggregation of particles of a medication, wherein amount of a medication particles smaller than 10 µm is suitable.

Therefore, the present invention provides a single-dose dry powder inhaler, which can be adjusted for application with any medication. Thus, there is no need to produce a plurality of inhalers each dedicated for a different medication. Moreover, providing replaceable modules of a mouthpiece assembly having different geometries or an integrated mouthpiece assembly having different geometries allows to use a single inhaler for inhaling different medications without having to have a plurality of dedicated inhalers.

As described above in detail, a dry-powder inhaler is operable within an entire useful range of resistivity of known dry-powder inhalers. Below a method for selecting a mouthpiece assembly for an inhaler according to the invention will be described for a reference medication.

A reference medication is here understood to mean a medication having a specific composition, for which, based on models, a certain particle size distribution has been determined, in particular, for active substance particles, wherein said distribution will potentially be most beneficial therapeutically, that is, the so-called originator, or a medication having a specific composition and a specific particle distribution, in particular, active substance particle distribution, wherein said particle distribution has been tested for therapeutic properties and has been granted a marketing authorization.

In the first stage, a mouthpiece assembly is selected, either integrated or mouthpiece modules, as described above, comprising air intake passages 25 of the mouthpiece base 21B and a mouthpiece tube 21C having specific geometry, characterized by a specific self-resistance coefficient R_{D} and which will later constitute a reference inhaler in the process of selecting a suitable inhaler. Such a reference inhaler is subjected to tests to determine particle size distribution of a medication in an aerosol of said medication, generated using a reference inhaler and, if necessary, deposition of particular fractions of the medication in a patient's airways.

In the second stage, the test results are compared against the data for a reference medication to determine if the obtained particle size values are correct relative to the reference medication. If the test results for the reference inhaler are incorrect, one determines if the particle size values obtained for the reference inhaler are bigger or smaller than those for the reference medication.

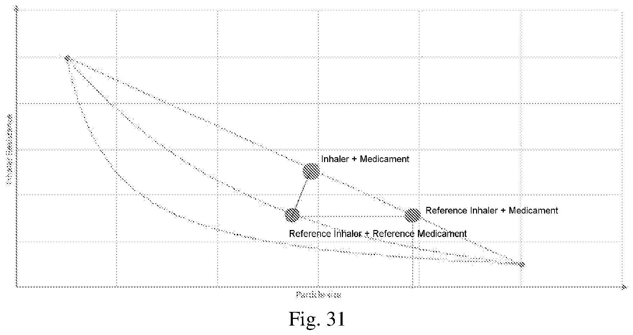

In such a case, in the third stage, a different mouthpiece assembly is selected, comprising air intake passages 25 and a mouthpiece tube 21C having different, specific geometries. If the particles of an aerosolized medication are bigger than those for the reference medication, then a mouthpiece assembly is selected comprising air intake passages 25 of the mouthpiece base 21B and a mouthpiece tube 21C such that the self-resistance coefficient R_{D1} of the next inhaler is greater than self-resistance R_{D} of the reference inhaler. If the particles of an aerosolized medication were smaller than those for the reference medication, then a mouthpiece assembly is selected comprising air intake passages 25 of the mouthpiece base 25 and a mouthpiece tube 21C such that the self-resistance coefficient R_{D1} of the next inhaler is smaller than self-resistance R_{D} of the reference inhaler. The above-described method for selecting the next inhaler is shown illustratively in Fig. 30 and 31, respectively.

Where between a reference inhaler and another inhaler having a lower or higher self-resistance coefficient R_{D1} there is a possibility to select a mouthpiece assembly having geometrical parameters, which, theoretically, can yield particle size distribution results similar to those of the reference medication and ensure a better deposition of particular fractions in a patient's airways, then tests should be carried out for the inhaler comprising a mouthpiece assembly having such parameters. If the test results are similar to those of the reference medication, it should be concluded that such an inhaler with the mouthpiece assembly having given geometrical parameters of the air intake passages 25 of the mouthpiece base and the mouthpiece tube 21C is the inhaler with the target mouthpiece assembly that will be dedicated for a specific formulation.

As described above, the method of the invention thus allows to adjust an inhaler for a medication allowing to obtain an aerosol of the medication having a desired particle distribution, in particular, active substance particle distribution, preferably smaller than 10 µm. Use of the inhaler according to the invention eliminates the need to provide a plurality of separate inhalers dedicated for specific medications that need to be administered in the form of an aerosol containing particles characterized by a specific particle size distribution of a medication, especially of an active substance.

The inhaler of the invention is described herein for inhaling a medication in the form of a capsule. However, the inhaler of the invention may be used for inhaling a medication in the form of a portion placed in the capsule chamber 14. In such a case, the inhaler does not comprise the push-buttons for perforating a capsule.

The term "medication" as used herein is understood to mean a pharmaceutical composition, also referred to as a medicine, containing at least one active substance and additional and auxiliary substances suitable for the composition for inhaling in the form of a dry powder, especially a carrier. The present invention does not relate directly to a medication, hence the issue of a medicinal composition used in the inhaler of the invention will not be discussed herein in detail.

The features indicated in the above-described embodiments of the invention, especially the preferred ones, can be combined or replaced in any given way and in any given combination, whereby all new connections or combinations possible are deemed to be fully disclosed in the description of the present invention, provided that they do not contain mutually conflicting features.

The invention was described above by means of preferred embodiments by way of example only. Based on the above disclosure a specialist in the field will recognize that modifications, variants or equivalents are possible that fall within the inventive intention of the present invention without exceeding the scope of the appended claims.

### Reference numbers:

- 1: body of the inhaler
- 2: supporting pillar
- 3: latch
- 4: body rib
- 5: body rib
- 6: lower guide of the body
- 7: push-button of the inhaler
- 8: upper protrusion of the push-button
- 9: lower protrusion of the push-button
- 10: spikes
- 11: chamber element
- 12: recess
- 13: guide hole for the spikes 10
- 14: capsule chamber
- 15: upper cover of the inhaler
- 16: hinge groove of the upper cover
- 17: latch of the upper cover
- 18: latch spot of the upper cover
- 19: upper guide in the upper cover of the chamber
- 20: protrusion of the upper cover
- 21: modular mouthpiece of the inhaler
- 21A: mouthpiece mesh of the inhaler
- 21B: mouthpiece base of the inhaler
- 21C: mouthpiece tube of the inhaler
- 22: cover of the tube module
- 23: spring for pushing back the push-buttons
- 24: latch
- 25: air intake passage for inhalation
- 26: rotation chamber of the inhaler
- 27: mouthpiece tube constriction
- 28: latch spot of mouthpiece base
- 29: tube latches
- 30: recess of the chamber element
- S1: internal diameter of the mouthpiece tube
- S2: through diameter of the mouthpiece tube
- H: height of the air intake passage
- W: width of the air intake passage

## Claims

1. A single-dose dry powder inhaler, comprising
a base assembly comprising a chamber (14) configured to receive a medication; and
a mouthpiece assembly comprising
air intake passages (25) for supplying air into the inhaler,
a rotation chamber (26) for generating an aerosol of the medication, wherein the rotation chamber (26) is in air communication with the air intake passages (25),
a tube (21C) comprising an internal passage in air communication with the rotation chamber (26) for discharging the aerosol of the medication and administering said aerosol to a patient;
wherein the base assembly and the mouthpiece assembly are configured such that the inhaler is capable of assuming an open configuration, in which access to the chamber (14) of the base assembly is provided for inserting therein the medication, and a closed configuration, in which the chamber (14) of the base assembly is in air communication with the rotation chamber (26) of the mouthpiece assembly for transferring the medication from the chamber (14) to the rotation chamber (26) for forming out of it the aerosol of the medication,
**characterized in that**
at least one of the air intake passages (25) and the tube (21C) of the mouthpiece assembly is selectable,
**and in that** the air intake passages (25) have a ratio of one of the transverse dimensions (H, W) to the other of the transverse dimensions (H, W), measured in a cross-section of said air intake passage (25), is in the range from 1:1 to 1:0.10 and the tube (21C) comprises a constriction (27) arranged in the internal passage of the tube such that the coverage of the passage is in the range from 0% to 95%.

2. An inhaler according to claim 1, **characterized in that** the chamber (14) is a capsule chamber adapted to receive a medication in the form of a capsule **and in that** it also comprises means for perforating a capsule in the capsule chamber.

3. An inhaler according to claim 1, **characterized in that** the air intake passages (25) have a ratio of one of the transverse dimensions (H, W) to the other of the transverse dimensions (H, W) in the range from 1:0.85 to 1:0.25.

4. An inhaler according to claim 1, **characterized in that** the coverage of the internal passage of the tube (21C) is in the range from 0% to 85%.

5. An inhaler according to claim 1, **characterized in that** the mouthpiece assembly is a replaceable, integrated module of the mouthpiece assembly.

6. An inhaler according to claim 1, **characterized in that** the mouthpiece assembly comprises a replaceable module of the air intake passages and a replaceable tube module.

7. A method for adjusting a single-dose dry powder inhaler for administering a specific medication **characterized in that,** said method comprises
providing a reference medication having a desired particle distribution of a medication,
providing a medication to be administered by means of an inhaler,
providing an inhaler according to any of the claims 1 to 6, comprising a mouthpiece assembly having the desired geometries of the air intake passage (25) and the tube (21C) of the mouthpiece assembly, for which the self-resistance coefficient (R_{D}) has a specific value,
generating an aerosol of the medication by means of the inhaler and analyzing said aerosol to determine the particle size distribution of the medication,
if the particles of the medication in an aerosol generated by the inhaler are bigger than those of the reference medication, another mouthpiece assembly is selected comprising air intake passages (25) of the mouthpiece base (21B) and a mouthpiece tube (21C) such that the self-resistance coefficient (R_{D}) of the inhaler with another mouthpiece assembly is bigger than the self-resistance (R_{D}) of the inhaler with the prior mouthpiece assembly,
however, if the particles of the medication in an aerosol generated by the inhaler are smaller than those of the reference medication, another mouthpiece assembly is selected comprising air intake passages (25) of the mouthpiece base (21B) and a mouthpiece tube (21C) such that the self-resistance coefficient (R_{D}) of the inhaler with another mouthpiece assembly is bigger than the self-resistance (R_{D}) of the inhaler with the prior mouthpiece assembly,
and again, an aerosol is generated by means of the inhaler with another mouthpiece assembly and the generated aerosol is analyzed to determine the particle size distribution of the medication,
the above stages being repeated until the particle distribution of the medication in an aerosol generated by the inhaler with the mouthpiece assembly having a selected geometry of the air intake passages (25) of the mouthpiece base (21B) and the mouthpiece tube (21C) corresponds with the particle distribution of the reference medication within the range of ±15% of the desired value of particle size distribution, and the inhaler with such a mouthpiece assembly is deemed to be the inhaler dedicated for the medication, unless the inhaler with the first selected mouthpiece assembly meets the above condition.

8. A method for adjusting an inhaler according to claim 7, **characterized in that** a change in the geometry of the air intake passages (25) of the mouthpiece base (21B) and/or of the mouthpiece tube (21C) is effected by replacing the integrated mouthpiece assembly.

9. A method for adjusting an inhaler according to claim 8, **characterized in that** a change in the geometry of the air intake passages (25) of the mouthpiece base (21B) and/or of the mouthpiece tube (21C) is effected by replacing at least one of the air intake passage module and the tube module.

## Patentansprüche

1. Einzeldosis-Trockenpulverinhalator, umfassend
eine Basisanordnung, die eine Kammer (14) umfasst, die zur Aufnahme eines Medikaments konfiguriert ist; und
eine Mundstückanordnung, umfassend
Lufteinlasskanäle (25) zum Zuführen von Luft in den Inhalator,
eine Rotationskammer (26) zum Erzeugen eines Aerosols des Medikaments, wobei die Rotationskammer (26) in Luftverbindung mit den Lufteinlasskanälen (25) steht,
ein Rohr (21C), das einen inneren Kanal in Luftverbindung mit der Rotationskammer (26) zum Ausstoßen des Aerosols des Medikaments und zum Verabreichen des Aerosols an einen Patienten umfasst;
wobei die Basisanordnung und die Mundstückanordnung so konfiguriert sind, dass der Inhalator eine offene Konfiguration, in der ein Zugang zur Kammer (14) der Basisanordnung zum dortigen Einführen des Medikaments bereitgestellt wird, und eine geschlossene Konfiguration annehmen kann, in der die Kammer (14) der Basisanordnung in Luftverbindung mit der Rotationskammer (26) der Mundstückanordnung zum Überführen des Medikaments von der Kammer (14) zur Rotationskammer (26) zur Bildung des Aerosols des Medikaments aus dieser steht,
**dadurch gekennzeichnet, dass**
mindestens einer der Lufteinlasskanäle (25) und das Rohr (21C) der Mundstückanordnung auswählbar ist,
**und dass** die Lufteinlasskanäle (25) ein Verhältnis von einer der Querabmessungen (H, W) zu der anderen der Querabmessungen (H, W), gemessen in einem Querschnitt des Lufteinlasskanals (25), im Bereich von 1:1 bis 1:0,10 aufweisen und das Rohr (21C) eine Verengung (27) umfasst, die in dem inneren Kanal des Rohrs so angeordnet ist, dass die Abdeckung des Kanals im Bereich von 0 % bis 95 % liegt.

2. Inhalator nach Anspruch 1, **dadurch gekennzeichnet, dass** die Kammer (14) eine Kapselkammer ist, die zur Aufnahme eines Medikaments in Form einer Kapsel angepasst ist, **und dass** er auch Mittel zum Perforieren einer Kapsel in der Kapselkammer umfasst.

3. Inhalator nach Anspruch 1, **dadurch gekennzeichnet, dass** die Lufteinlasskanäle (25) ein Verhältnis von einer der Querabmessungen (H, W) zu der anderen der Querabmessungen (H, W) im Bereich von 1:0,85 bis 1:0,25 aufweisen.

4. Inhalator nach Anspruch 1, **dadurch gekennzeichnet, dass** die Abdeckung des inneren Kanals des Rohrs (21C) im Bereich von 0 % bis 85 % liegt.

5. Inhalator nach Anspruch 1, **dadurch gekennzeichnet, dass** die Mundstückanordnung ein austauschbares, integriertes Modul der Mundstückanordnung ist.

6. Inhalator nach Anspruch 1, **dadurch gekennzeichnet, dass** die Mundstückanordnung ein austauschbares Modul der Lufteinlasskanäle und ein austauschbares Rohrmodul umfasst.

7. Verfahren zum Einstellen eines Einzeldosis-Trockenpulverinhalators zur Verabreichung eines bestimmten Medikaments, **dadurch gekennzeichnet, dass** das Verfahren Folgendes umfasst:
Bereitstellen eines Referenzmedikaments mit einer gewünschten Partikelverteilung eines Medikaments,
Bereitstellen eines Medikaments, das mittels eines Inhalators verabreicht werden soll,
Bereitstellen eines Inhalators nach einem der Ansprüche 1 bis 6, der eine Mundstückanordnung mit den gewünschten Geometrien des Lufteinlasskanals (25) und des Rohrs (21C) der Mundstückanordnung umfasst, für die der Eigenwiderstandskoeffizient (R_{D}) einen bestimmten Wert aufweist,
Erzeugen eines Aerosols des Medikaments mittels des Inhalators und Analysieren des Aerosols, um die Partikelgrößenverteilung des Medikaments zu bestimmen,
wenn die Partikel des Medikaments in einem durch den Inhalator erzeugten Aerosol größer sind als die des Referenzmedikaments, eine andere Mundstückanordnung ausgewählt wird, die Lufteinlasskanäle (25) der Mundstückbasis (21B) und ein Mundstückrohr (21C) umfasst, sodass der Eigenwiderstandskoeffizient (R_{D}) des Inhalators mit einer anderen Mundstückanordnung größer ist als der Eigenwiderstand (R_{D}) des Inhalators mit der vorherigen Mundstückanordnung,
jedoch, wenn die Partikel des Medikaments in einem durch den Inhalator erzeugten Aerosol kleiner sind als die des Referenzmedikaments, eine andere Mundstückanordnung ausgewählt wird, die Lufteinlasskanäle (25) der Mundstückbasis (21B) und ein Mundstückrohr (21C) umfasst, sodass der Eigenwiderstandskoeffizient (R_{D}) des Inhalators mit einer anderen Mundstückanordnung größer ist als der Eigenwiderstand (R_{D}) des Inhalators mit der vorherigen Mundstückanordnung,
und wiederum ein Aerosol mittels des Inhalators mit einer anderen Mundstückanordnung erzeugt wird und das erzeugte Aerosol analysiert wird, um die Partikelgrößenverteilung des Medikaments zu bestimmen,
wobei die obigen Schritte wiederholt werden, bis die Partikelverteilung des Medikaments in einem Aerosol, das von dem Inhalator mit der Mundstückanordnung erzeugt wird, die eine ausgewählte Geometrie der Lufteinlasskanäle (25) der Mundstückbasis (21B) und des Mundstückrohrs (21C) aufweist, mit der Partikelverteilung des Referenzmedikaments innerhalb des Bereichs von ±15 % des gewünschten Werts der Partikelgrößenverteilung übereinstimmt, und der Inhalator mit einer solchen Mundstückanordnung als der für das Medikament dedizierte Inhalator gilt, es sei denn, der Inhalator mit der ersten ausgewählten Mundstückanordnung erfüllt die obige Bedingung.

8. Verfahren zum Einstellen eines Inhalators nach Anspruch 7, **dadurch gekennzeichnet, dass** eine Änderung der Geometrie der Lufteinlasskanäle (25) der Mundstückbasis (21B) und/oder des Mundstückrohrs (21C) durch Auswechseln der integrierten Mundstückanordnung bewirkt wird.

9. Verfahren zum Einstellen eines Inhalators nach Anspruch 8, **dadurch gekennzeichnet, dass** eine Änderung der Geometrie der Lufteinlasskanäle (25) der Mundstückbasis (21B) und/oder des Mundstückrohrs (21C) durch Auswechseln von mindestens einem von dem Lufteinlasskanalmodul und dem Rohrmodul bewirkt wird.

## Revendications

1. Inhalateur à poudre sèche à dose unique, comprenant
un ensemble de base comprenant une chambre (14) configurée pour recevoir un médicament; et
un ensemble d'embout buccal comprenant
des passages d'admission d'air (25) pour amener de l'air dans l'inhalateur,
une chambre de rotation (26) pour générer un aérosol du médicament, dans lequel la chambre de rotation (26) est en communication d'air avec les passages d'admission d'air (25),
un tube (21C) comprenant un passage interne en communication d'air avec la chambre de rotation (26) pour décharger l'aérosol du médicament et administrer ledit aérosol à un patient;
dans lequel l'ensemble de base et l'ensemble d'embout buccal sont configurés de telle sorte que l'inhalateur est capable de prendre une configuration ouverte, dans laquelle un accès à la chambre (14) de l'ensemble de base est prévu pour y insérer le médicament, et une configuration fermée, dans laquelle la chambre (14) de l'ensemble de base est en communication d'air avec la chambre de rotation (26) de l'ensemble d'embout buccal pour transférer le médicament de la chambre (14) à la chambre de rotation (26) pour en former l'aérosol du médicament,
**caractérisé en ce que**
au moins l'un des passages d'admission d'air (25) et du tube (21C) de l'ensemble embout buccal peut être sélectionné,
**et en ce que** les passages d'admission d'air (25) ont un rapport de l'une des dimensions transversales (H, W) à l'autre des dimensions transversales (H, W), mesurées dans une section transversale dudit passage d'admission d'air (25), est dans la plage de 1:1 à 1:0.10 et le tube (21C) comprend un étranglement (27) disposé dans le passage interne du tube de telle sorte que le recouvrement du passage est dans la plage de 0% à 95%.

2. Inhalateur selon la revendication 1, **caractérisé en ce que** la chambre (14) est une chambre à capsule adaptée pour recevoir un médicament sous la forme d'une capsule **et en ce qu'il** comprend également des moyens pour perforer une capsule dans la chambre à capsule.

3. Inhalateur selon la revendication 1, **caractérisé en ce que** les passages d'admission d'air (25) ont un rapport de l'une des dimensions transversales (H, W) à l'autre des dimensions transversales (H, W) dans la plage de 1:0.85 à 1:0.25.

4. Inhalateur selon la revendication 1, **caractérisé en ce que** le recouvrement du passage interne du tube (21C) est dans la plage de 0% à 85%.

5. Inhalateur selon la revendication 1, **caractérisé en ce que** l'ensemble d'embout buccal est un module intégré, remplaçable, de l'ensemble d'embout buccal.

6. Inhalateur selon la revendication 1, **caractérisé en ce que** l'ensemble d'embout buccal comprend un module remplaçable des passages d'admission d'air et un module de tube remplaçable.

7. Méthode pour ajuster un inhalateur à poudre sèche à dose unique pour administrer un médicament spécifique, **caractérisée en ce que** ladite méthode comprend
la fourniture d'un médicament de référence ayant une distribution de particules souhaitée d'un médicament,
la fourniture d'un médicament à administrer au moyen d'un inhalateur,
la fourniture d'un inhalateur selon l'une quelconque des revendications 1 à 6, comprenant un ensemble d'embout buccal ayant les géométries souhaitées du passage d'admission d'air (25) et du tube (21C) de l'ensemble d'embout buccal, pour lequel le coefficient d'auto-résistance (RD) a une valeur spécifique,
la génération d'un aérosol du médicament au moyen de l'inhalateur et l'analyse dudit aérosol pour déterminer la distribution de taille des particules du médicament,
si les particules du médicament dans un aérosol généré par l'inhalateur sont plus grosses que celles du médicament de référence, un autre ensemble d'embout buccal est sélectionné comprenant des passages d'admission d'air (25) de la base d'embout buccal (21B) et un tube d'embout buccal (21C) de sorte que le coefficient d'auto-résistance (RD) de l'inhalateur avec un autre ensemble d'embout buccal est plus grand que l'auto-résistance (RD) de l'inhalateur avec l'ensemble d'embout buccal précédent,
si cependant les particules du médicament dans un aérosol généré par l'inhalateur sont plus petites que celles du médicament de référence, un autre ensemble d'embout buccal est sélectionné comprenant des passages d'admission d'air (25) de la base d'embout buccal (21B) et un tube d'embout buccal (21C) de sorte que le coefficient d'auto-résistance (RD) de l'inhalateur avec un autre ensemble d'embout buccal est plus grand que l'auto-résistance (RD) de l'inhalateur avec l'ensemble d'embout buccal précédent,
et encore, un aérosol est généré au moyen de l'inhalateur avec un autre ensemble d'embout buccal et l'aérosol généré est analysé pour déterminer la distribution de taille des particules du médicament,
les étapes ci-dessus étant répétées jusqu'à ce que la distribution de particules du médicament dans un aérosol généré par l'inhalateur avec l'ensemble d'embout buccal ayant une géométrie sélectionnée des passages d'admission d'air (25) de la base d'embout buccal (21B) et du tube d'embout buccal (21C) corresponde à la distribution de particules du médicament de référence dans la plage de ±15% de la valeur souhaitée de distribution de taille de particules, et l'inhalateur avec un tel ensemble d'embout buccal est considéré comme étant l'inhalateur dédié au médicament, à moins que l'inhalateur avec le premier ensemble d'embout buccal sélectionné ne satisfasse à la condition ci-dessus.

8. Méthode pour ajuster un inhalateur selon la revendication 7, **caractérisée en ce qu'un** changement de la géométrie des passages d'admission d'air (25) de la base d'embout buccal (21B) et/ou du tube d'embout buccal (21C) est effectuée en remplaçant l'ensemble d'embout buccal intégré.

9. Méthode pour ajuster un inhalateur selon la revendication 8, **caractérisée en ce qu'un** changement de la géométrie des passages d'admission d'air (25) de la base d'embout buccal (21B) et/ou du tube d'embout buccal (21C) est effectuée en remplaçant au moins l'un du module de passage d'admission d'air et du module de tube.
